(19) Europäisches Patentamt
European Patent Office
Office européen des brevets

(11) EP 4 810 957 A1

(12) **EUROPEAN PATENT APPLICATION**
published in accordance with Art. 153(4) EPC

(43) Date of publication:
**23.09.2026 Bulletin 2026/39**

(21) Application number: **24891712.2**

(22) Date of filing: **07.11.2024**

(51) International Patent Classification (IPC):
***G01N 33/50*** *(2006.01)*

(52) Cooperative Patent Classification (CPC):
**G01N 33/50**

(86) International application number:
**PCT/KR2024/017554**

(87) International publication number:
**WO 2025/105766 (22.05.2025 Gazette 2025/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.11.2023 KR 20230159475**
**13.12.2023 KR 20230181155**

(71) Applicant: **SK Bioscience Co., Ltd.**
**Gyeonggi-do 13494 (KR)**

(72) Inventors:
- **LEE, Hye Won**
  **Seongnam-si, Gyeonggi-do 13494 (KR)**
- **KIM, Tae Won**
  **Seongnam-si, Gyeonggi-do 13494 (KR)**
- **LEE, Jeong Min**
  **Seongnam-si, Gyeonggi-do 13494 (KR)**

(74) Representative: **Weickmann & Weickmann PartmbB**
**Postfach 860 820**
**81635 München (DE)**

# (54) METHOD FOR DETERMINING THE HEXOSAMINE CONTENT IN PNEUMOCOCCAL POLYSACCHARIDE SAMPLE SOLUTION

(57) The present disclosure relates to a method for determining a hexosamine content in a polysaccharide sample solution. In quantitatively evaluating whether the hexosamine content in a produced pneumococcal polysaccharide undiluted solution sample is suitable for use in a vaccine, the method according to an aspect provides accurate assay compared to the hexosamine content test method described in the European Pharmacopoeia, and thus can be effectively utilized for quality control of hexosamine-containing pneumococcal polysaccharide vaccines or hexosamine-containing polysaccharide-protein conjugate vaccines.

EP 4 810 957 A1

## Description

### Technical Field

The present disclosure relates to a method for determining a hexosamine content in a pneumococcal polysaccharide sample solution.

### Background Art

*Streptococcus pneumonia* is a type of the *Streptococcus* that is Gram-positive and hemolytic, and is a major cause of meningitis, pneumonia, and severe invasive infectious diseases in infants, children, and the elderly worldwide. At least 1.6 million people die every year from pneumococcal disease, and the incidence of invasive pneumococcal infectious disease is particularly high in children aged 5 or less and adults aged 65 or more, who have weakened immune systems. *S. pneumonia* may be classified into more than approximately 90 serotypes based on the structural and immunological characteristics of capsular polysaccharide, which is a virulence factor surrounding the outside (cell membrane) of the *S. pneumonia***,** and some of these serotypes have been reported to cause invasive diseases**.** A total of 37 serotypes were identified in invasive *S. pneumonia* collected from 1996 to 2008, and vaccines such as polysaccharide vaccines and polysaccharide-protein conjugate vaccines have been manufactured by using the capsular polysaccharide of *S. pneumonia* of these identified serotypes.

Meanwhile, the accuracy of vaccine quality assessment methods has a direct impact on vaccine safety, and the European Pharmacopoeia (EP) provides methods for quantitatively determining contents of monosaccharides, such as hexosamine, in pneumococcal polysaccharide vaccines. However, issues have been raised that such hexosamine content test method for polysaccharide vaccines described in the EP did not meet the serotype-specific hexosamine content compliance criteria set forth in the EP and the WHO guidelines, even when proceeding a test using working standard samples. As a result, there is a growing need for evaluation methods that can more accurately test the hexosamine content.

[Prior Art Documents]

[Non-Patent Documents]

(Non-Patent Document 1) EUROPEAN PHARMACOPOEIA 11.0, 2.5.20. Hexosamines in polysaccharide vaccine, 2022

### Disclosure of Invention

### Technical Problem

An aspect provides a method for determining a hexosamine content in a pneumococcal polysaccharide sample solution, the method including: (a) treating a glucosamine standard solution at a series of concentrations according to a predetermined procedure and plotting a standard curve by measuring absorbance; and (b) treating a polysaccharide sample solution according to a predetermined procedure, and by measuring absorbance, calculating a hexosamine content in the polysaccharide sample solution based on the standard curve obtained in (a), wherein the predetermined procedure includes: adding a hydrochloric acid solution to each test tube containing a standard solution or the polysaccharide sample solution to proceed hydrolysis; adding a sodium carbonate solution to proceed neutralization; adding an acetylacetone solution to proceed a reaction; adding ethanol to proceed precipitation; adding a dimethylaminobenzaldehyde (DMAB) solution as a color-developing agent to proceed derivatization and dissolve the precipitate; and measuring absorbance.

### Solution to Problem

An aspect provides a method for determining a hexosamine content in a pneumococcal polysaccharide sample solution, the method including: (a) treating a glucosamine standard solution at a series of concentrations according to a predetermined procedure and plotting a standard curve by measuring absorbance; and (b) treating a polysaccharide sample solution according to a predetermined procedure, and by measuring absorbance, calculating a hexosamine content in the polysaccharide sample solution based on the standard curve obtained in (a), wherein the predetermined procedure includes: adding a hydrochloric acid solution to each test tube containing a standard solution or the polysaccharide sample solution to proceed hydrolysis; adding a sodium carbonate solution to proceed neutralization;

adding an acetylacetone solution to proceed a reaction; adding ethanol to proceed precipitation; adding a dimethylaminobenzaldehyde (DMAB) solution as a color-developing agent to proceed derivatization and dissolve the precipitate; and measuring absorbance.

The term "hexosamine" refers to an amino sugar with a molecular formula of $C_6H_{13}NO_5$ formed by adding an amino group to a hexose, and includes galactosamine, glucosamine, fructosamine, and mannosamine.

The term "standard solution" refers to a solution containing a substance of known concentration that is used to plot a calibration curve when quantitatively analyzing a concentration of a target substance by using spectrophotometry. The method according to an aspect was confirmed to satisfy the hexosamine content compliance criteria set forth in the EP and the WHO guidelines for all serotypes of working standards, regardless of whether a standard substance, glucosamine, was acetylated (Experimental Example 2.3). In an embodiment, the standard solution containing glucosamine may be an N-acetyl D-glucosamine solution or a D-(+)-glucosamine hydrochloride solution. In an embodiment, the series of concentrations falls within a range of 0 to 250 μg/mL, and the number of the series of concentrations is at least eight. For example, the series of concentrations may include 0, 25, 62.5, 125, 150, 175, 200, and 225 μg/mL, but is not limited thereto, and sequential concentrations within the range above may be selected and used.

The term "polysaccharide sample solution" may be used interchangeably with the term "test solution", and refers to a sample solution containing an analyte whose concentration is to be measured. For example, to determine whether an undiluted capsular polysaccharide solution, which is obtained through processes including cell lysis, isolation, and purification from a culture medium of a strain producing specific pneumococcal serotypes, is suitable for use in a vaccine, the method may involve analysis using, as the polysaccharide sample solution, a test sample of an undiluted solution of purified pneumococcal capsular polysaccharide or a diluted solution thereof. In an embodiment, the polysaccharide sample solution may be a solution in which a sample has been diluted 10 to 15 times. When the dilution factor of the polysaccharide sample solution is too high beyond the range above or too low falling below the range above, the absorbance of a sample may fall outside the absorbance range of the standard solution depending on the pneumococcal serotypes, and thus the assay methods described herein may not be suitable for use with all pneumococcal serotypes.

In an embodiment, the pneumococcal serotype may be any serotype containing hexosamine, and specifically, may be any one selected from the group consisting of 4, 5, 9N, 9V, 10A, 12F, 14, 15B, 19A, and 19F.

Hereinafter, the predetermined procedure will be described in detail.

Hydrolysis:

This process involves adding a hydrochloric acid solution as a hydrolysis buffer to each test tube containing either a glucosamine standard solution prepared at a series of concentrations or a prepared polysaccharide sample solution, to induce a reaction, and is intended to hydrolyze and expose specific residues within sugars.

In an embodiment, the concentration of the hydrochloric acid solution may be 12.0±0.5 M, for example, 12.0±0.4 M, 12.0±0.3 M, 12.0±0.2 M, 12.0±0.1 M, 12.0±0.05 M, or 12.0±0.01 M. When the concentration of the hydrochloric acid solution is too high beyond the range above, excessive evaporation of hydrochloric acid may occur during the reaction at room temperature, causing difficulties in carrying out a normal hydrolysis reaction. Upon the reaction at low temperatures, not only specific target residues but also other residues may be exposed, and the polysaccharide itself may break down, making it impossible to perform an assay method. In addition, when the concentration of the hydrochloric acid solution is too low falling below the range above, the polysaccharide residues may not be sufficiently exposed to combine with a color-developing reagent. Thus, in the process of adding a color-developing reagent such as DMAB later, the reaction intensity may be too week to perform assay on polysaccharides.

In an embodiment, the volume ratio of the hydrochloric acid solution to the standard solution or the polysaccharide sample solution may be 2:0.5 to 1.5, for example, 2:0.5 to 1.3, 2:0.5 to 1.1, 2:0.5 to 1.0, 2:0.7 to 1.5, 2:0.7 to 1.3, 2:0.7 to 1.1, 2:0.7 to 1.1, 2:0.9 to 1.5, 2:0.9 to 1.3, 2:0.9 to 1.1, 2:0.9 to 1.0, 2:1.0 to 1.5, or 2:1.0 to 1.3. When the volume of the hydrochloric acid solution added is too large beyond the range above or too small falling below the range above, the concentration of the hydrochloric acid solution at the start of hydrolysis is too high or too low, causing the aforementioned problems.

In an embodiment, the hydrolysis may involve reacting for 4 hours to 6 hours, for example, 4 hours to 5 hours, or 5 hours to 6 hours. When the hydrolysis reaction time is too long beyond the range above, excessive hydrolysis may occur, damaging the polysaccharides and accordingly failing to proceed an assay method normally. When the hydrolysis reaction time is too short falling below the range above, hydrolysis may not proceed sufficiently, resulting in insufficient exposure of the residues within the polysaccharide and accordingly failing to proceed an assay method normally. The hydrolysis may be carried out by heating, and may be carried out at temperatures of 100 °C or higher, for example 100 °C to 150 °C, 100 °C to 120 °C, or 130 °C to 150 °C.

In an embodiment, the test tube may be made of glass, and considering that the thermal conductivity may vary depending on the test tube material, the reaction temperature and the reaction time may be based on a glass-type test tube.

Following the hydrolysis reaction, cooling each solution at room temperature may be performed.

Neutralization and titration of hydrolysate:

This process involves neutralizing the solutions in each test tube that became acidic after the hydrolysis by adding a base thereto. Specifically, this process involves adding, for the purpose of neutralization, a sodium carbonate ($Na_2CO_3$) solution to each test tube that has undergone the hydrolysis reaction, and then proceeding a reaction by adding an acetylacetone solution to make color changes more distinct in a subsequent color reaction using a DMAB solution.

In an embodiment, the concentration of the sodium carbonate solution may be 2.0±1.0 M, for example, 2.0±0.9 M, 2.0±0.8 M, 2.0±0.7 M, 2.0±0.6 M, 2.0±0.5 M, 2.0±0.4 M, 2.0±0.3 M, 2.0±0.2 M, 2.0±0.1 M, 2.0±0.05 M, or 2.0 ±0.01 M.

In an embodiment, after the hydrolysis reaction, the volume ratio of the sodium carbonate solution with respect to the total volume of the solutions in each test tube may be 3:3.5 to 4.5, for example, 3:3.5 to 4.3, 3:3.5 to 4.1, 3:3.5 to 4.0, 3:3.7 to 4.5, 3:3.7 to 4.3, 3:3.7 to 4.1, 3:3.7 to 4.0, 3:3.9 to 4.5, 3:3.9 to 4.3, 3:3.9 to 4.1, 3:3.9 to 4.0, 3:4.0 to 4.5, or 3:4.0 to 4.3.

In an embodiment, the concentration of the acetylacetone solution may be 0.2±0.1 M, for example, 0.2±0.09 M, 0.2±0.08 M, 0.2±0.07 M, 0.2±0.06 M, 0.2±0.05 M, 0.2±0.04 M, 0.2±0.03 M, 0.2±0.02 M, 0.2 to 0.3 M, or 0.216 M.

In an embodiment, after the addition of the sodium carbonate solution, the volume ratio of the acetylacetone solution with respect to the total volume of the solutions in each test tube may be 7:4.5 to 5.5, for example, 7:4.5 to 5.3, 7:4.5 to 5.1, 7:4.5 to 5.0, 7:4.7 to 5.5, 7:4.7 to 5.3, 7:4.7 to 5.1, 7:4.7 to 5.0, 7:4.9 to 5.5, 7:4.9 to 5.3, 7:4.9 to 5.1, 7:4.9 to 5.0, 7:5.0 to 5.5, or 7:5.0 to 5.3.

In an embodiment, the proceeding of the reaction by adding the acetylacetone solution may be carried at temperatures of 100 °C or higher, for example, 100 °C to 150 °C, 100 °C to 120 °C, or 130 °C to 150 °C, for 20 minutes to 40 minutes, for example, 20 minutes to 35 minutes, 20 minutes to 30 minutes, 20 minutes to 25 minutes, 25 minutes to 40 minutes, 25 minutes to 35 minutes, 25 minutes to 30 minutes, 30 minutes to 40 minutes, 30 minutes to 35 minutes, or 35 minutes to 40 minutes.

Following the proceeding of the reaction by adding the acetylacetone solution, cooling each solution at room temperature may be performed.

Color development:

This process involves: adding ethanol to each neutralized solution to precipitate the polysaccharides in which reactive groups capable of undergoing a reaction with a reagent have been sufficiently exposed by the hydrolysis in the precious process; adding a dimethylaminobenzaldehyde (DMAB) solution as a color-developing agent to dissolve the precipitate; and measuring absorbance.

In an embodiment, after the addition of the acetylacetone solution, the volume ratio of the ethanol with respect to the total volume of the solutions in each test tube may be 1.2:0.5 to 1.5, for example, 1.2:0.5 to 1.5, 1.2:0.5 to 1.3, 1.2:0.5 to 1.1, 1.2:0.5 to 1.0, 1.2:0.7 to 1.5, 1.2:0.7 to 1.3, 1.2:0.7 to 1.1, 1.2:0.7 to 1.1, 1.2:0.9 to 1.5, 1.2:0.9 to 1.3, 1.2:0.9 to 1.1, 1.2:0.9 to 1.0, 1.2:1.0 to 1.5, or 1.2:1.0 to 1.3. When the volume of the ethanol solution added is too small falling below the range above, precipitation may not occur sufficiently, resulting in difficulties to achieve a normal reaction.

In an embodiment, the concentration of the DMAB solution may be 0.2±0.1 M, for example, 0.2±0.09 M, 0.2 ±0.08 M, 0.2±0.07 M, 0.2±0.06 M, 0.2±0.05 M, 0.2±0.04 M, 0.2±0.03 M, 0.2 to 0.3 M, or 0.223 M. When the concentration of the DMAB solution is too high beyond the range above, economic efficiency may be reduced, whereas, when the concentration is too low falling below the range above, the color reaction may not occur sufficiently, causing measurement to be impossible.

In an embodiment, after addition of the ethanol, the volume ratio of the DMAB solution with respect to the total volume of the solutions in each test tube may be 2.2:0.1 to 1.0, for example, 2.2:0.1 to 0.8, 2.2:0.1 to 0.5, 2.2:0.3 to 1.0, 2.2:0.3 to 0.8, 2.2:0.3 to 0.5, 2.2:0.5 to 1.0, or 2.2:0.5 to 0.8. When the volume of the DMAB solution added is too high beyond the range above, the concentration of the polysaccharide sample solution may become excessively diluted, resulting in inaccurate absorbance measurement, whereas, when the concentration is too low falling below the range above, the color reaction may not occur sufficiently, causing measurement to be impossible.

In an embodiment, the adding of the DMAB solution to dissolve the precipitate may involve: adding the DMAB solution, followed by vortexing the resulting solution for 10 seconds to 30 seconds; and resting the solution at room temperature for 5 minutes to 15 minutes, for example, 5 minutes to 10 minutes, 8 minutes, 7 minutes, or 5 minutes. When the resting time is too long beyond the range above, testing convenience may be reduced since the color development stage requires a tester to continuously monitor color changes with the naked eyes, and the sample itself may decompose, causing difficulties in accurate absorbance measurement. When the resting time is too short falling below the range above, the color reaction may not occur sufficiently, also causing difficulties in absorbance measurement. The method has the

advantage of improving testing convenience, as absorbance measurement may be achieved in a short resting time.

The absorbance measurement may involve measuring absorbance at a wavelength of 530 nm by using a UV-Vis spectrophotometer on the solutions in each test tube that have undergone the resting.

In the case of the hexosamine content test method described in the EP (referred to as EP method), despite changes in various test conditions, such as hydrolysis concentrations (8 M, 10 M, and 12 M HCl), hydrolysis time (1, 2, 3, and 4 hours), materials of test tube for analysis (PP tube or glass tube), color reactions (inverting or vortexing), and acetylation of standard substances, it was confirmed that the working standard sample did not meet the serotype-specific hexosamine content compliance criteria set forth in the EP and WHO guidelines (Experimental Example 1). Meanwhile, regardless of acetylation of the standard substance, the hexosamine content test method of the present disclosure was found to stably exceed the hexosamine content compliance criteria set forth in the EP and the WHO guidelines for all serotypes of the working standard (Experimental Example 2), demonstrating excellent accuracy in assay performance, and thus may be effectively used in the manufacture of a safe pneumococcal polysaccharide vaccine.

## Advantageous Effects of Invention

The method according to an embodiment allows, in quantitatively evaluating whether a hexosamine content in a produced pneumococcal polysaccharide undiluted solution sample is suitable for use in a vaccine, more accurate assay compared to the hexosamine content test method described in the European Pharmacopoeia, and thus may be effectively used for quality control of hexosamine-containing pneumococcal polysaccharide vaccines for each serotype or hexosamine-containing polysaccharide-protein conjugate vaccines.

## Mode for the Invention

Hereinafter, the present disclosure will be described in more detail through Examples. However, these Examples are provided to illustrate the present disclosure, and the scope of the present invention is not limited to these Examples.

## Experimental Example 1. EP method and modification test

To evaluate whether the quality of samples of a produced pneumococcal polysaccharide undiluted solution is suitable for use in a vaccine, the assay performance was evaluated for the hexosamine content test method for polysaccharide vaccines described in the EP (hereinafter referred to as EP method) and a method under modified test conditions on this basis. Specifically, a sample from ATCC company as the working standard and a sample provided by the applicant company were used to evaluate whether these samples satisfied the hexosamine content criteria set forth in the EP and the WHO guidelines.

### 1.1 Hexosamine content assay method according to EP method (Comparative Example)

#### 1.1.1 Preparation of D-(+)-glucosamine hydrochloride standard solution

0.6 g of D-(+)-glucosamine hydrochloride was weighed and added to 50 mL of triple-distilled water, and the mixed solution was vortexed until sufficiently dissolved, and then diluted to prepare 10 mg/mL D-(+)-glucosamine stock solution. 2.5 mL of 10 mg/mL D-(+)-glucosamine stock solution was added to a 50 mL conical tube, and triple-distilled water was added thereto to have a final volume of 50 mL. The resulting solution was sufficiently mixed by vortexing to prepare a 500 μg/mL D-(+)-glucosamine standard solution. The 500 μg/mL D-(+)-glucosamine standard solution was added to a tube and diluted to the concentrations shown in Table 1 to prepare standard solutions and blank.

[Table 1]

| Division | Concentration (μg/mL) | 500 μg/mL D-(+)-glucosamine standard solution (μL) | Triple-distilled water (μL) | Total volume (μL) |
|---|---|---|---|---|
| Blank | 0 | 0 | 1000 | 1000 |
| STD 1 | 125 | 250 | 750 | 1000 |
| STD 2 | 250 | 500 | 500 | 1000 |
| STD 3 | 375 | 750 | 250 | 1000 |
| STD 4 | 500 | 1000 | 0 | 1000 |

### 1.1.2 Preparation of test solution

In Table 2, the hexosamine content compliance criteria (%content ratio) for each serotype are shown, as specified in EP Volume 1. Vaccine Section Table 0966-1 (Percentage Contents of Components of Monovalent Bulk Polysaccharides). Based on the %content ratio, the sample was appropriately diluted so that the hexosamine concentration fell within the standard solution concentration range of 125 to 500 μg/mL. The sample was prepared in a tube so that the final volume of the diluted sample was 1 mL.

[Table 2]

| Serotype | %content ratio | Serotype | %content ratio |
|---|---|---|---|
| **4** | ≥ 40 | **12F** | ≥ 25 |
| **5** | ≥ 20 | **14** | ≥ 20 |
| **9N** | ≥ 28 | **15B** | ≥ 15 |
| **9V** | ≥ 13 | **19A** | ≥ 12 |
| **10A** | ≥ 12 | **19F** | ≥ 12.5 |

Specifically, samples were diluted appropriately in consideration of the minimum dilution factor and the maximum dilution factors for each serotype. When the hexosamine content of the diluted sample was beyond the concentration range of the standard solution, further dilution was performed without being limited to the minimum dilution factor and the maximum dilution factor. When the dilution factor was at least 10-fold, serial dilution was performed. Here, the maximum dilution factor and the minimum dilution factor for the samples were calculated according to the following formula:

Maximum dilution factor = dry weight value per serotype (mg/mL) x %content ratio for serotype / 125 μg/mL

Minimum dilution factor = dry weight value per serotype (mg/mL) x %content ratio for serotype / 500 μg/mL

### 1.1.3 Hydrolysis

Polysaccharide hydrolysis was performed by adding a hydrolysis buffer to the blank, the D-(+)-glucosamine standard solutions at various concentrations, and the test solutions prepared in Sections 1.1.1 and 1.1.2. Specifically, 1 mL of 8 M HCl solution was added to tubes containing 1 mL of each of the blank, the D-(+)-glucosamine standard solutions at various concentrations, and the test solutions prepared in Sections 1.1.1 and 1.1.2, and the resulting solutions were vortexed. The tube caps were closed, and the solutions were allowed to react for 1 hour in a water bath set to 90 to 99 °C, following by sufficient cooling at room temperature for 15 minutes.

Here, the 8 M HCl solution was prepared by adding 131.53 mL of 37 % HCl to a 250 mL glass bottle, filling the bottle with triple-distilled water to achieve the final volume of 200 mL, and then stirring the resulting solution thoroughly using a magnetic stirrer.

### 1.1.4 Neutralization and titration of hydrolysate

A base was added to each solution, which had become acidic due to the hydrolysis, to proceed titration. Specifically, 0.05 mL of a thymolphthalein solution (5 g thymolphthalein /1 L EtOH) was added to each tube cooled to room temperature, and the resulting solutions were vortexed. Next, a 200 g/L NaOH solution was slowly added until the color of the blank, the standard solution, and the test solution changed from colorless to blue, and then a 1M HCl solution was added for titration until the blue color disappeared and the solutions turned colorless. Triple-distilled water was added to each tube to achieve the final volume of 10 mL, and the resulting solutions were vortexed.

From 10 mL of each of the neutralized blank, standard solution, and test solution, only 1 mL of each solution was transferred to a new tube, and 1 mL of an acetylacetone solution (1 volume acetylacetone/50 volume $Na_2CO_3$) was added thereto. The resulting solutions were sufficiently vortexed. The tube caps were closed, and the solutions were allowed to react for 45 minutes in a water bath set to 90 °C, following by sufficient cooling at room temperature for 15 minutes.

Here, the thymolphthalein solution was prepared by weighing 0.05 of thymolphthalein and adding it to 10 mL of ethanol, and vortexing the resulting solution until sufficiently dissolved. Here, the 200 g/L NaOH solution was prepared by weighing 40 g of sodium hydroxide and adding it to a 250 mL glass bottle, filling the bottle with triple-distilled water to achieve the final volume of 200 mL, and then stirring the resulting solution thoroughly using a magnetic stirrer. A $Na_2CO_3$

solution was prepared by weighing 2.65 g of $Na_2CO_3$ and adding it to 50 mL of triple-distilled water, and then vortexing the resulting solution until sufficiently dissolved. The acetylacetone solution was prepared by adding 0.5 mL of acetylacetone to 25 mL of the $Na_2CO_3$ solution, and then sufficiently mixing the resulting solution by vortexing.

**1.1.5 Color development**

2.5 mL of ethanol was added to each tube cooled to room temperature, and the resulting solution was vortexed thoroughly to ensure the formation of a precipitate. 1 mL of a DMAB solution (0.8 g dimethylaminobenzaldehyde /15 mL EtOH + 15 mL HCl) was added to each tube, which was then inverted to dissolve the precipitate thoroughly. 4.5 mL of ethanol was added to each tube to achieve the final volume of 10 mL, and the tubes were inverted. After resting the solutions at room temperature for 90 minutes in a light-shielding condition, the absorbance was measured at a wavelength of 530 nm using a UV-Vis spectrophotometer. A calibration curve was plotted using the absorbance values of the measured standard solution, and on this basis, the hexosamine content was quantified from the absorbance values of the measured test solution.

Here, the DMAB solution was a 0.179 M DMAB solution prepared by weighing 0.8 g of 4-(dimetylamino)-benzaldehyde and mixing with 15 mL of ethanol and 15 mL of 37 % HCl by vortexing until sufficiently dissolved.

**1.2 Evaluation based on hydrolysis concentration and time in EP method**

To determine whether the HCl concentration and reaction time during the hydrolysis in the EP method described in Section 1.1 are factors affecting the hexosamine content measurement, serotypes for which the pass/fail determination could vary due to changes in the test method were used, and the hexosamine content of each serotype sample was measured under hydrolysis conditions of 8 M, 10 M, and 12 M HCl for 1, 2, and 3 hours. The results are shown in Table 3. For serotypes 4 and 12F, it was confirmed that, during the pre-test, the hexosamine content was lower in the working standard sample provided by ATCC company than in the polysaccharide undiluted solution provided by the applicant company. Thus, these serotypes were set as worst cases and regarded as control materials in the test.

Here, the 10 M HCl solution was prepared by adding 164.41 mL of 37 % HCl to a 250 mL glass bottle, filling the bottle with triple-distilled water to achieve the final volume of 200 mL, and then stirring the resulting solution thoroughly using a magnetic stirrer. The 12 M HCl solution was a 37 % HCl stock solution.

[Table 3]

| Serotype | Lot No. | 8 M HCl | | | 10 M HCl | | | 12 M HCl | | | Standard (%) |
|---|---|---|---|---|---|---|---|---|---|---|---|
| | | 1 hr | 2 hr | 3 hr | 1 hr | 2 hr | 3 hr | 1 hr | 2 hr | 3 hr | |
| 4 | A04042 201C | 30 | 34 | 29 | 35 | 32 | 34 | 37 | 33 | 32 | ≥ 40 |
| | ATCC | 23 | 26 | 26 | 26 | 24 | 26 | 31 | 29 | 27 | |
| 12F | CTAA1 242 | 28 | 26 | 27 | 24 | 25 | 25 | 30 | 31 | 28 | ≥ 25 |
| | ATCC | 21 | 21 | 22 | 21 | 22 | 21 | 24 | 23 | 24 | |
| 19A | A96042 301F | 13 | 14 | 15 | 12 | 15 | 15 | 16 | 17 | 18 | ≥ 12 |
| 19F | CTAA9242 | 12 | 11 | 13 | 13 | 14 | 15 | 14 | 16 | 17 | ≥ 12.5 |

As a result, as shown in Table 3, serotypes 19A and 19F were confirmed to be suitable for use in a vaccine by satisfying the assay criteria when heated in the 12 M HCl for 3 hours. However, although all serotypes were used as working standards in the assay method, serotypes 4 and 12F did not satisfy the assay criteria, confirming that the assay method was not performed normally.

**1.3 Evaluation based on test tube material in EP method**

Considering that the temperature available for the water bath in the test method described in Section 1.1 was high such as 90 °C or higher, a comparative experiment was conducted using different test tube materials (polypropylene (PP) tube vs glass tube) to determine whether the thermal conductivity of the test tubes affects the hexosamine content measurement. The hexosamine content of serotype 4 was measured under conditions of hydrolysis with 8 M, 10 M, and 12 M HCl for 4 hours, and the results are shown in Table 4. In the present test, a test for comparing materials was conducted by fixing the time to 4 hours to confirm whether the test tubes remained visually intact even at a hydrolysis reaction time longer than the 3-hour condition in Table 6, and at the same time, whether the hexosamine content increased after 3 hours or

more.

[Table 4]

| Serotype | Lot No. | Tube | 8 M | 10 M | 12 M | Standard (%) |
|---|---|---|---|---|---|---|
| 4 | A04042201C | Polypropylene | 31 | 33 | 35 | ≥ 40 |
| | | Glass | 39 | 36 | 36 | |

As a result, as shown in Table 4, it was confirmed that the use of glass tubes resulted in a greater hexosamine content than the use of PP tubes. Expecting some differences in the sample-to-test tube interactions and the thermal conductivity to occur to some extent, it was confirmed that the test tubes needed to be replaced with glass tubes, and thus subsequent experiments were conducted using glass tubes.

## 1.4 Evaluation based on precipitate-dissolving method in EP method

In the test method described in Section 1.1, a DMAB solution was added to the precipitate formed upon the addition of ethanol after the second water bath reaction, and then, the tube was inverted to dissolve the precipitate and observed for any color reaction. To determine whether the degree of color development varies depending on the precipitate-dissolving method, vortexing was additionally performed for comparison. Both inverting and vortexing during the color development stage were performed for 10 to 30 seconds, and the hexosamine contents were measured from samples of serotypes 4, 9N, 12F, 19A, and 19F under the hydrolysis condition of 8 M HCl for 1 hour. The results are shown in Table 5.

[Table 5]

| Serotype | Lot No. | Inverting | Vortexing | Standard (%) |
|---|---|---|---|---|
| 4 | A04042201C | 32 | 36 | ≥ 40 |
| 9N | CTAA9145J | 30 | 31 | ≥ 28 |
| 12F | CTAA1242 | 27 | 27 | ≥ 25 |
| 19A | A96042301F | 11 | 12 | ≥ 12 |
| 19F | CTAA97402 | 12 | 12 | ≥ 12.5 |

As a result, as shown in Table 5, compared to inverting, vortexing resulted in hexosamine contents that were equal to or higher in all serotypes, confirming that vortexing is effective for dissolution of the precipitate more thoroughly. In addition, inverting has been previously used to prevent damage to polysaccharides, but in a suspension prepared using a vortexing technique, the precipitate could be also effectively dissolved without damaging polysaccharides. Therefore, subsequent experiments were conducted using a vortexing technique to dissolve the precipitate during the color development stage.

## 1.5 Evaluation based on acetylation of standard substance in EP method

As confirmed in Sections 1.2 to 1.4, despite various modifications to the EP Method, changing the test conditions and the test tube materials was insufficient even for the ATCC sample, which is the working standard, to stably exceed the hexosamine content criteria set forth in the EP and the WHO guidelines. Table 6 shows the ratio of acetylated hexosamine to non-acetylated hexosamine by serotypes. Based on the finding from the batch results that the serotypes not adequately detected by the existing EP hexosamine assay methods commonly had a relatively high proportion of mainly acetylated hexosamine (Table 6), it was aimed to confirm the quantitative results depending on whether the standard substance.

[Table 6]

| Hexosamine | | Serotype | | | | | | | | | |
|---|---|---|---|---|---|---|---|---|---|---|---|
| | | 4 | 5 | 9N | 9V | 10A | 12F | 14 | 15B | 19A | 19F |
| Glucosamine | Ac * | 0 | 0 | 1 | 0 | 0 | 0 | 1 | 1 | 0 | 0 |
| | - | 0 | 2 | 3 | 3 | 0 | 2 | 1 | 1 | 1 | 1 |

(continued)

| Hexosamine | | Serotype | | | | | | | | | |
|---|---|---|---|---|---|---|---|---|---|---|---|
| | | **4** | **5** | **9N** | **9V** | **10A** | **12F** | **14** | **15B** | **19A** | **19F** |
| **Galactos-amine** | **Ac** | 1 | 0 | 0 | 0 | 4 | 1 | 0 | 0 | 0 | 0 |
| | **-** | 1 | 0 | 0 | 1 | 1 | 1 | 2 | 3 | 0 | 0 |
| **Mannos-amine** | **Ac** | 1 | 0 | 1 | 1 | 0 | 1 | 0 | 0 | 1 | 1 |
| | **-** | 0 | 0 | 0 | 0 | 0 | 0 | 0 | 0 | 0 | 0 |
| **Fucos-amine** | **Ac** | 1 | 1 | 0 | 0 | 0 | 1 | 0 | 0 | 0 | 0 |
| | **-** | 0 | 0 | 0 | 0 | 0 | 0 | 0 | 0 | 0 | 0 |
| **Pneumos-amine** | **Ac** | 0 | 1 | 0 | 0 | 0 | 0 | 0 | 0 | 0 | 0 |
| | **-** | 0 | 0 | 0 | 0 | 0 | 0 | 0 | 0 | 0 | 0 |
| **Ratio of acetylated to non-acetylated hexosa-mine** | | 3/4 | 2/4 | 2/5 | 1/5 | 1/5 | 3/6 | 1/4 | 1/5 | 1/2 | 1/2 |
| | | **0.75** | **0.50** | **0.40** | **0.20** | **0.20** | **0.50** | **0.25** | **0.20** | **0.50** | **0.50** |
| (* Ac: acetylated) | | | | | | | | | | | |

Specifically, unlike existing unacetylated hexosamine (D-(+)-glucosamine hydrochloride, Formula 1), acetylated hexosamine (N-acetyl D-glucosamine, Formula 2) was used as the standard substance, and a comparative test was conducted based on whether the standard substance was acetylated. The hydrolysis time was set to the two extremes among the previously tested conditions (minimum 1 hour and maximum 4 hours) and based on the results in Section 1.4, the experiment was conducted by adding the DMAB solution and vortexing the resulting solution to proceed the color reaction. The hexosamine content was measured by the EP method using N-acetyl D-glucosamine as the standard substance, and the results are shown in Table 7. When using such an acetylated standard substance, a molecular weight thereof is greater than that of an existing standard substance, and thus the quantitative results also increase arithmetically simply by the difference in the molecular weight. Therefore, to access only the improvement effect on reactivity depending on the acetylation, the measured content results were multiplied by the molecular weight ratio of the standard substances (179.17/221.21, about 81 %) to offset the molecular weight of the acetyl group, and the recalculated results are shown in Table 7. Meanwhile, regarding the HCl molecule in Formula 1, since it is a salt bonded to the glucosamine molecule, which is a target to be quantified, and is therefore ionized upon dissolution, its molecular weight (36.46) is excluded from the concentration of the standard solution. Therefore, the HCl molecule is not a matter of concern when comparing the structures of the two standard substances. Here, the N-acetyl D-glucosamine standard solution was prepared by dissolving 500 mg of N-acetyl D-glucosamine in 50 mL of triple-distilled water to create a 10 mg/mL stock solution, which was then diluted 20-fold to a concentration of 500 μg/mL for use as the standard solution.

[Formula 1]

D-(+)-glucosamine hydrochloride (M.W.: 179.17)

[Formula 2]

N-acetyl D-glucosamine (M.W.: 221.21)

[Table 7]

| Serotype | Lot No. | 8 M HCl | | | | Standard (%) |
|---|---|---|---|---|---|---|
| | | 1 hr | 4 hr | Recalculated | | |
| | | | | 1 hr | 4 hr | |
| 4 | A04042201C | 47 | 45 | 38 | 36 | ≥ 40 |
| | ATCC | 43 | 46 | 35 | 37 | |
| 9N | CTAA9145J | 41 | 42 | 33 | 34 | ≥ 28 |
| 12F | CTAA1242 | 42 | 35 | 34 | 28 | ≥ 25 |
| | ATCC | 32 | 32 | 26 | 26 | |
| 19A | A96042301F | 16 | 19 | 13 | 15 | ≥ 12 |
| 19F | CTAA97402 | 15 | 20 | 12 | 16 | ≥ 12.5 |

As a result, as shown in Table 7, in comparison with the results in Table 5 based on the recalculated values, the improvement resulting from the changes in the standard substances was minimal. Even when using the ATCC sample as the working standard, the hexosamine content still did not stably exceed the hexosamine content criteria set forth in the EP and the WHO guidelines.

**Experimental Example 2. SK method and modification test**

Despite various modifications, it was confirmed that, using the existing EP method, even working standards for the serotypes being analyzed did not meet the hexosamine content criteria set forth in the EP and the WHO guidelines. Accordingly, a hexosamine content assay method (hereinafter referred to as SK method) as described in Section 2.1 was established and used to evaluate whether the working standards for the serotypes being analyzed satisfy the hexosamine content criteria for polysaccharide vaccines set forth in the EP and the WHO guidelines.

## 2.1 Hexosamine content assay method according to SK method (Example)

### 2.1.1 Preparation of N-acetyl D-glucosamine standard solution

125 mg of N-acetyl D-glucosamine was weighed and added to a 500 mL beaker and triple-distilled water was added thereto to achieve the final volume of 500 mL. The resulting solution was sufficiently stirred using a magnetic stirrer, so as to prepare a 250 μg/mL N-acetyl D-glucosamine standard solution. The 250 μg/mL N-acetyl D-glucosamine standard solution was added to a glass tube and diluted to the concentrations shown in Table 8 to prepare standard solutions and blank.

[Table 8]

| Division | Concentration (μg/mL) | 250 μg/mL N-acetyl D-glucosamine standard solution (μL) | Triple-distilled water (μL) | Total volume (μL) |
|---|---|---|---|---|
| Blank | 0 | 0 | 200 | 200 |
| STD 1 | 25 | 20 | 180 | 200 |
| STD 2 | 62.5 | 50 | 150 | 200 |
| STD 3 | 125 | 100 | 100 | 200 |
| STD 4 | 150 | 120 | 80 | 200 |
| STD 5 | 175 | 140 | 60 | 200 |
| STD 6 | 200 | 160 | 40 | 200 |
| STD 7 | 225 | 180 | 20 | 200 |

### 2.1.2 Preparation of test solution

Test solutions were diluted appropriately as shown in Table 9, and only 0.2 mL of the final volume was added in a glass tube.

[Table 9]

| Dilution factor | Sample (μL) | Distilled water (μL) | Total volume (μL) |
|---|---|---|---|
| 1/10 | 60 | 540 | 600 |
| 1/15 | 40 | 560 | 600 |
| 1/20 | 30 | 570 | 600 |

### 2.1.3 Hydrolysis

Hydrolysis was performed by adding a hydrolysis buffer to the blank, the N-acetyl D-glucosamine standard solutions at various concentrations, and the test solutions prepared in Sections 2.1.1 and 2.1.2. Specifically, 0.1 mL of 12 M HCl solution was added to tubes containing 0.2 mL of each of the blank, the N-acetyl D-glucosamine standard solutions at various concentrations, and the test solutions prepared in Sections 2.1.1 and 2.1.2, and the resulting solutions were vortexed. The glass tube caps were closed, and the solutions were allowed to react for 4 hours in a water bath set to 100 °C, following by sufficient cooling at room temperature for 15 minutes.

Here, the 12 M HCl solution was a 37 % HCl stock solution.

### 2.1.4 Neutralization and titration of hydrolysate

A base was added to each solution, which had become acidic due to the hydrolysis, to proceed neutralization. Specifically, 0.4 mL of a 2 M $Na_2CO_3$ solution was added to each glass tube cooled to room temperature, and the resulting solutions were vortexed while carefully monitoring carbon dioxide gas being released. Next, 0.5 mL of an acetylacetone solution was added to each glass tube, and the resulting solutions were vortexed. The glass tube caps were closed, and the solutions were allowed to react for 20 minutes in a water bath set to 100 °C, following by sufficient cooling at room

temperature for 15 minutes.

Here, the 2 M $Na_2CO_3$ solution was prepared by weighing 53 g of $Na_2CO_3$ and adding it to a 250 mL bottle, filling the bottle with triple-distilled water to achieve the final volume of 250 mL, and then stirring the resulting solution thoroughly using a magnetic stirrer. The acetylacetone solution was prepared by adding 0.4 mL of acetylacetone to 15 mL of the 2 M $Na_2CO_3$ solution, adding triple-distilled water thereto to achieve the final volume of 20 mL, and then sufficiently mixing the resulting solution by vortexing.

**2.1.5 Color development**

1 mL of ethanol was added to each glass tube cooled to room temperature, and the resulting solution was vortexed thoroughly to ensure the formation of a precipitate. 0.5 mL of a DMAB solution (Ehrlich's reagent) was added to each glass tube, and the resulting solutions were vortexed while carefully monitoring carbon dioxide gas being released. After resting at room temperature for 5 minutes, the absorbance was measured at a wavelength of 530 nm using a UV-Vis spectrophotometer. A calibration curve was plotted using the absorbance values of the measured standard solution, and on this basis, the hexosamine content was quantified from the absorbance values of the measured test solution.

Here, the DMAB solution (Ehrlich's reagent) was a 0.223 M DMAB solution prepared by weighing 1 g of p-dimethylaminobenzaldehyde, adding 15 mL of 95 % ethanol thereto, vortexing the resulting solution thoroughly, and then adding 15 mL of 37 % HCl and vortexing the resulting solution again thoroughly.

**2.2 Evaluation of hexosamine content analysis by SK method at different dilution factors of samples**

Analysis was performed on the same serotypes as in Experimental Example 1, by using the SK method using the acetylated standard substance described in Section 2.1. The hexosamine contents at different dilution factors of the samples were analyzed according to the SK method, and the results are shown in Table 10. Cases where the absorbance of the samples at different dilution factors was beyond the absorbance range of the standard solution (STD range out) were marked with an asterisk (*).

[Table 10]

| Seroty pe | Lot No. | 2X | 5X | 10X | 15X | 20X | Stand ard (%) |
|---|---|---|---|---|---|---|---|
| 4 | A04042201 C | 26* | 51* | 56 | 58 | 58 | ≥ 40 |
| | ATCC | 45* | 52 | 55 | 55 | 57 | |
| 5 | E19P344 | 35* | 41* | 41 | 43 | 39 | ≥ 20 |
| 9N | CTAA9145 J | 36* | 45* | 48 | 48 | 50 | ≥ 28 |
| 9V | CTAA9242 | 24* | 25 | 26 | 24 | 25 | ≥ 13 |
| | ATCC | 22 | 22 | 21 | 19* | 19* | |
| 10A | CTAA1042 | 17* | 19 | 19 | 19 | 20 | ≥ 12 |
| 12F | CTAA1242 | 37* | 41 | 38 | 36 | 37 | ≥ 25 |
| | ATCC | 31* | 32 | 32 | 29 | 30 | |
| 14 | CTAA1441 B | 29* | 35 | 35 | 36 | 36 | ≥ 20 |
| 15B | 210708T4 | 14* | 22* | 24 | 24 | 27 | ≥ 15 |
| 19A | A96042301 F | 26* | 27 | 27 | 25 | 25* | ≥ 12 |
| | ATCC | 21 | 22 | 22 | 23 | 20* | |
| 19F | CTAA9740 2 | 24* | 26 | 28 | 25 | 25 | ≥ 12.5 |
| | ATCC | 21 | 22 | 20 | 21 | 20* | |

To confirm the results satisfying the absorbance range of the standard solution, experiments were conducted at various dilution factors. As shown in Table 10, when the dilution factor fell outside the range of 10X to 15X, there were some serotypes for which the standard absorbance could not be measured, resulting in instances where the present assay method could not be uniformly applied to all serotypes. Therefore, it was confirmed that the range of dilution factors commonly applicable to the hexosamine assay method was 10X to 15X.

The experiments were repeated three times at the confirmed dilution factors, and the results are shown in Table

11. When the hexosamine content was analyzed using the acetylated standard substance, the SK method satisfied the hexosamine content criteria set forth in the EP and the WHO guidelines for both the ATCC sample as the working standard and the purified pneumococcal polysaccharide undiluted sample. Furthermore, as shown in Table 7, the values recalculated to account for the effect of the increase in molecular weight also satisfied the criteria for all samples.

[Table 11]

| Seroty pe | Lot No. | 1st | 2nd | 3rd | Averag e | %CV | Stand ard (%) |
|---|---|---|---|---|---|---|---|
| 4 | A04042201 C | 58 | 53 | 57 | 56 | 5 | ≥ 40 |
| | ATCC | 57 | 47 | 49 | 51 | 10 | |
| 5 | E19P344 | 41 | 38 | 38 | 39 | 4 | ≥ 20 |
| 9N | CTAA9145 J | 48 | 47 | 47 | 47 | 1 | ≥ 28 |
| 9V | CTAA9242 | 26 | 26 | 25 | 26 | 2 | ≥ 13 |
| 10A | CTAA1042 | 19 | 20 | 20 | 20 | 3 | ≥ 12 |
| 12F | CTAA1242 | 38 | 34 | 36 | 36 | 6 | ≥ 25 |
| | ATCC | 32 | 27 | 32 | 30 | 10 | |
| 14 | CTAA1441 B | 35 | 34 | 36 | 35 | 3 | ≥ 20 |
| 15B | 210708T4 | 24 | 25 | 26 | 25 | 4 | ≥ 15 |
| 19A | A96042301 F | 27 | 23 | 27 | 26 | 9 | ≥ 12 |
| 19F | CTAA9740 2 | 28 | 25 | 29 | 27 | 8 | ≥ 12.5 |

## 2.3 Evaluation based on acetylation of standard substance in SK method

To determine whether the SK method also showed differences depending on the standard substance, additional experiments were conducted using D-(+)-glucosamine hydrochloride which is the standard substance used in the existing EP method. To establish reaction conditions prior to the repeated tests, a comparative analysis was conducted under conditions involving hydrolysis with 12 M HCl for 1 hour and 4 hours and 5-minute resting and 90-minute resting after addition of a DMAB solution, and the results are shown in Table 12. Here, the D-(+)-glucosamine hydrochloride standard solution was a 250 μg/mL D-(+)-glucosamine standard solution prepared by dissolving 150 mg of D-(+)-glucosamine hydrochloride in 500 mL of triple-distilled water, and was added to a glass tube and diluted to the concentrations shown in Table 8 to prepare standard solutions and blank.

[Table 12]

| Serotyp e | Lot No. | Hydrolysis 1 hr | | Hydrolysis 4 hr | | Standard (%) |
|---|---|---|---|---|---|---|
| | | 5 min | 90 min | 5 min | 90 min | |
| 4 | A04042201 C | 51 | 52 | 54 | 54 | ≥ 40 |
| 9N | CTAA9145J | 38 | 39 | 41 | 41 | ≥ 28 |
| 19A | A96042301 F | 13 | 14 | 21 | 21 | ≥ 12 |
| 19F | CTAA97402 | 15 | 16 | 24 | 24 | ≥ 12.5 |

The experiments were repeated three times on 10 serotypes using the method that yielded the highest hexosamine content in Table 12, specifically under conditions of hydrolysis for 4 hours and 5-minute resting after addition of a DMAB solution, and the results are shown in Table 13.

[Table 13]

| Seroty pe | Lot No. | 1st | 2nd | 3rd | Averag e | %CV | Stand ard (%) |
|---|---|---|---|---|---|---|---|
| 4 | A04042201 C | 54 | 45 | 51 | 48 | 9 | ≥ 40 |
| | ATCC | 42 | 47 | 45 | 45 | 6 | |

(continued)

| Seroty pe | Lot No. | 1st | 2nd | 3rd | Averag e | %CV | Stand ard (%) |
|---|---|---|---|---|---|---|---|
| 5 | E19P344 | 38 | 37 | 36 | 37 | 3 | ≥ 20 |
| 9N | CTAA9145 J | 41 | 40 | 39 | 40 | 3 | ≥ 28 |
| 9V | CTAA9242 | 22 | 21 | 21 | 21 | 3 | ≥ 13 |
| 10A | CTAA1042 | 17 | 17 | 15 | 16 | 7 | ≥ 12 |
| 12F | CTAA1242 | 33 | 30 | 34 | 32 | 7 | ≥ 25 |
| | ATCC | 31 | 31 | 26 | 29 | 10 | |
| 14 | CTAA1441 B | 29 | 29 | 28 | 29 | 2 | ≥ 20 |
| 15B | 210708T4 | 18 | 19 | 19 | 19 | 3 | ≥ 15 |
| 19A | A96042301 F | 21 | 22 | 20 | 21 | 5 | ≥ 12 |
| 19F | CTAA9740 2 | 24 | 23 | 23 | 23 | 3 | ≥ 12.5 |

As a result, as shown in Table 13, when the SK method was applied to the assay method used to measure the hexosamine content, it was confirmed that all serotypes, including the ATCC sample as the working standard, stably exceeded the hexosamine content compliance criteria set forth in the EP and the WHO guidelines, regardless of the type of the standard substance.

## Claims

**1.** A method for determining a hexosamine content in a pneumococcal polysaccharide sample solution, the method comprising:

(a) treating a glucosamine standard solution at a series of concentrations according to a predetermined procedure and plotting a standard curve by measuring absorbance; and
(b) treating a polysaccharide sample solution according to a predetermined procedure, and by measuring absorbance, calculating a hexosamine content in the polysaccharide sample solution based on the standard curve obtained in (a),

wherein the predetermined procedure comprises:

adding a hydrochloric acid solution to each test tube containing the standard solution or the polysaccharide sample solution to proceed hydrolysis;
adding a sodium carbonate solution to proceed neutralization;
adding an acetylacetone solution to proceed a reaction;
adding ethanol to proceed precipitation;
adding a dimethylaminobenzaldehyde (DMAB) solution as a color developing agent to proceed derivatization and dissolve a precipitate; and
measuring absorbance.

**2.** The method of claim 1, wherein the glucosamine standard solution is an N-acetyl D-glucosamine solution or a D-(+)-glucosamine hydrochloride solution.

**3.** The method of claim 1, wherein the series of concentrations falls within a range of 0 to 250 μg/mL, and the number of the series of concentrations is at least eight.

**4.** The method of claim 1, wherein a concentration of the hydrochloric acid solution is 12.0±0.5 M.

**5.** The method of claim 1, wherein the hydrolysis is performed for 4 hours to 6 hours.

**6.** The method of claim 1, wherein a volume ratio of the hydrochloric acid solution to the standard solution or the polysaccharide sample solution is 2:0.5 to 1.5.

7. The method of claim 1, wherein a concentration of the DMAB solution is 0.2±0.1 M.

8. The method of claim 1, wherein the adding of the DMAB solution to dissolve the precipitate comprises adding the DMAB solution, followed by vortexing and resting 5 minutes to 15 minutes.

9. The method of claim 1, wherein the test tube is formed of a glass material.

10. The method of claim 1, wherein the polysaccharide sample solution is a solution in which a sample has been diluted 10-fold to 15-fold.

11. The method of claim 1, wherein the pneumococcal serotype is any one selected from the group consisting of 4, 5, 9N, 9V, 10A, 12F, 14, 15B, 19A, and 19F.

**INTERNATIONAL SEARCH REPORT**

International application No. **PCT/KR2024/017554**

**A. CLASSIFICATION OF SUBJECT MATTER**

**G01N 33/50**(2006.01)i

According to International Patent Classification (IPC) or to both national classification and IPC

**B. FIELDS SEARCHED**

Minimum documentation searched (classification system followed by classification symbols)

G01N 33/50(2006.01); C08B 37/00(2006.01); C12N 1/18(2006.01); C12P 19/26(2006.01); G01N 21/31(2006.01); G01N 21/78(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 폐렴구균(Streptococcus pneumoniae), 다당류(polysaccharide), 핵소사민(hexosamine), 글루코사민(glucosamine), 흡광도(absorbance), 표준곡선(standard curve), 염산(hydrochloric acid), 탄산나트륨(sodium carbonate), 아세틸아세톤(acetylacetone), 에탄올(ethanol), 디메틸아미노벤즈알데하이드(dimethylaminobenzaldehyde)

**C. DOCUMENTS CONSIDERED TO BE RELEVANT**

| Category* | Citation of document, with indication, where appropriate, of the relevant passages | Relevant to claim No. |
|---|---|---|
| Y | ZABLACKIS, Earl et al. Chapter 7 Bacterial Polysaccharide Vaccines: Analytical Perspectives. Springer-Verlag Berlin Heidelberg. 2015. [NUNNALLY, B.K. et al. (eds.). Vaccine Analysis: Strategies, Principles, and Control]. pp. 271-299. DOI 10.1007/978-3-662-45024-6_7. See pages 276-278; and tables 7.2 and 7.4. | 1-11 |
| Y | KR 10-0543692 B1 (HAN, Man Deuk) 20 January 2006 (2006-01-20) See paragraphs [0080]-[0081]. | 1-11 |
| A | KR 10-2022-0075374 A (BIOLOGICAL E LIMITED) 08 June 2022 (2022-06-08) See entire document. | 1-11 |
| A | SOBOCINSKI, Philip Z. et al. Improved Continuous-Flow Method for Determination of Total Serum Hexosamines. Clinical Chemistry. 1976, vol. 22, no. 8, pp. 1394-1396. See entire document. | 1-11 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 February 2025** | **20 February 2025** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

INTERNATIONAL SEARCH REPORT

International application No.
PCT/KR2024/017554

C. DOCUMENTS CONSIDERED TO BE RELEVANT

| Category* | Citation of document, with indication, where appropriate, of the relevant passages | Relevant to claim No. |
|---|---|---|
| A | CN 105695350 B (TIBET TIANHONG SCIENCE & TECHNOLOGY CO., LTD.) 07 February 2020 (2020-02-07)<br>See entire document. | 1-11 |

Form PCT/ISA/210 (second sheet) (July 2022)

**INTERNATIONAL SEARCH REPORT**
**Information on patent family members**

International application No.
**PCT/KR2024/017554**

| Patent document cited in search report | Publication date (day/month/year) | Patent family member(s) | Publication date (day/month/year) |
|---|---|---|---|
| KR 10-0543692 B1 | 20 January 2006 | KR 10-2005-0036495 A | 20 April 2005 |
| KR 10-2022-0075374 A | 08 June 2022 | CN 114450576 A | 06 May 2022 |
| | | EP 4038369 A1 | 10 August 2022 |
| | | EP 4038369 A4 | 01 November 2023 |
| | | US 2022-0390423 A1 | 08 December 2022 |
| | | WO 2021-064748 A1 | 08 April 2021 |
| CN 105695350 B | 07 February 2020 | CN 105695350 A | 22 June 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Hexosamines in polysaccharide vaccine. *EUROPEAN PHARMACOPOEIA*, 2022, vol. 11 (0), 2.5.20
- Vaccine Section Table 0966-1 (Percentage Contents of Components of Monovalent Bulk Polysaccharides. *EP*, vol. 1